# EUROPEAN PATENT APPLICATION

(11) **EP 2 860 547 A1**
(43) Date of publication of application: **15.04.2015**
(21) Application number: 14188024.5
(22) Date of filing: 08.10.2014
(51) Int. Cl.: G01S 7/52, G01S 15/89, G10K 11/34

(54) **Apparatus and method for beamforming**

(30) Priority: 08.10.2013 KR 20130120028
(71) Applicant: Samsung Electronics Co., Ltd, Gyeonggi-do 443-742 (KR)
(72) Inventor: Kim, Bae Hyung, Yongin-si, Gyeonggi-do (KR); Kim, Young Il, Suwon-si Gyeonggi-do (KR); Song, Jong Keun, Yongin-si, Gyeonggi-do (KR); Lee, Seung Heun, Seongnam-si, Gyeonggi-do (KR); Cho, Kyung Il, Seoul (KR)
(74) Representative: Grünecker Patent- und Rechtsanwälte PartG mbB

(57) **Abstract**

Disclosed herein is a beamforming apparatus (200) for beamforming signals which are transmitted or received by a probe (100) which includes a plurality of transducer blocks (110, 120, 130, 140), each of which includes a plurality of transducers (111 - 143). The apparatus (200) includes a controller (210) configured to control components to delay signals to be transmitted by the plurality of transducers (111 - 143) included in each transducer block (110, 120, 130, 140) or signals received by the plurality of transducers (111 - 143) included in each transducer block (110, 120, 130, 140), and an analog beamformer (300) which includes a plurality of beamforming units (310, 320, 330, 340) which correspond to the respective transducer blocks (110, 120, 130, 140). The plurality of beamforming units (310, 320, 330, 340) is configured to perform analog beamforming on signals transmitted or received by the plurality of transducers (111 - 143) included in each transducer block (110, 120, 130, 140) based on a control signal which is received from the controller (210), to reduce a dynamic delay range, thereby reducing a size of a delay line.

## Description

### BACKGROUND

### 1. Field

Exemplary embodiments relate to an apparatus and method for beamforming.

### 2. Description of the Related Art

In general, a probe of an ultrasonic diagnosis apparatus is formed as a transducer. Upon being transmitted to a specific part inside a patient body by a probe of an ultrasonic diagnosis apparatus, ultrasonic waves of several kiloHertz (kHz) to several hundreds of megaHertz (MHz) are partially reflected from layers between various different tissues. In particular, ultrasonic waves are reflected by a part inside the human body, in which a density change occurs, for example, blood cells in blood plasma, small structures in organs, etc. The reflected ultrasonic waves cause oscillations in the transducer of the probe, and the transducer outputs electrical pulses as a result of the vibrations. The electrical pulses are converted into an image. However, a reflected ultrasonic signal typically has very low intensity and a low signal-to-noise ratio (SNR). Thus, there is a need for technologies for increasing the intensity and SNR of the reflected ultrasonic signal in order to convert the ultrasonic signal into image information. One of these technologies is beamforming.

Here, the beamforming refers to a technology for strengthening signal intensity by superposing signals via a plurality of transducers when a plurality of transducers is used for transmission and reception of the signals. A concept of transmission/reception beamforming using a one-dimensional transducer will now be described. A point at which image information is acquired is referred to as a focal point. In this regard, a plurality of transducers is typically arranged in a straight line, and thus distances between the transducers and the focal point are different. Accordingly, because an amount of time taken to transmit a signal from each transducer and to return the signal after being reflected varies, received signals do not have matched phases upon being superposed, and thus are not amplified. As a result, there is a need for a procedure for matching non-matched phases of signals in order to perform beamforming.

A method of matching phases is performed by delaying transmitted and received signals, and may be classified as any of several methods based on the method of delaying the signals. Beamforming is generally classified into analog beamforming and digital beamforming. The analog beamforming is performed by delaying a signal using a circuit device, whereas the digital beamforming entails digitizing and storing a signal and then reading data after a predetermined period of time elapses.

### SUMMARY

Therefore, it is an aspect of one or more exemplary embodiments to provide an apparatus and method for beamforming, by which one-dimensional (1 D) or two-dimensional (2D) array transducers are divided into a plurality of blocks, delay profiles (i.e., delay times) are calculated for the respective blocks, and beamforming is performed on signals for each block based on the calculated delay profile to reduce a dynamic delay range (DDR), thereby reducing a size of a delay line (i.e., an overall size of analog beamformer).

Additional aspects of the exemplary embodiments will be set forth in part in the description which follows and, in part, will be obvious from the description, or may be learned by practice of the exemplary embodiments.

In accordance with one aspect of one or more exemplary embodiments, a beamforming apparatus for beamforming signals which are transmitted or received by a probe which includes a plurality of transducer blocks, each of which includes a respective plurality of transducers. The beamforming apparatus includes a controller configured to control components to delay signals to be transmitted by the plurality of transducers for each transducer block and signals received by the plurality of transducers for each transducer block, and an analog beamformer which includes a plurality of beamforming modules which correspond to the respective transducer blocks and which are configured to perform analog beamforming on signals which are transmitted or received by the corresponding plurality of transducers based on a control signal which is received from the controller.

The controller is further configured to control the components to calculate a delay profile for each of the plurality of transducer blocks, to calculate a delay time for each transducer with respect to each of the plurality of transducer blocks based on the corresponding calculated delay profiles, and to delay the signals to be transmitted and the received signals based on the calculated delay times.

Each of the plurality of transducers included in each of the plurality of transducer blocks may be one-dimensionally (1 D) arranged or two-dimensionally (2D) arranged.

The beamforming apparatus may further include a storage device configured to store a number of the transducer blocks and combinations of outputs of transducers included in each of the plurality of transducer blocks, wherein the transducers included in each of the plurality of transducer blocks are arranged to be adjacent to each other.

Each of the plurality of beamforming modules may include a signal delayer configured to delay signals to be transmitted by the plurality of transducers included in the corresponding transducer block and signals received by the plurality of transducers included in the corresponding transducer block, a switcher configured to perform a switching operation based on whether signals which are input into the signal delayer are the signals to be transmitted or the received signals, and an adder configured to add delayed signals which are output from the signal delayer when the signals which are input into the signal delayer are the received signals.

The signal delayer may include a plurality of delay lines configured to implement respective delay times based on the control signal.

The analog beamformer may further include an analog adder configured to add signals which are output from the respective adders included in the plurality of beamforming modules.

The beamforming apparatus may further include an analog-digital converter configured to convert an analog signal generated by the analog adder into a digital signal.

In accordance with another aspect of one or more exemplary embodiments, a beamforming apparatus for beamforming signals which are transmitted or received by two-dimensional (2D) transducers includes an analog beamformer configured to perform analog beamforming on signals which are transceived by a plurality of transducer blocks, each of the transducer blocks including a respective plurality of transducer sub-blocks which is arranged in a first direction, and each of the transducer sub-blocks including a respective plurality of transducers, and a digital beamformer configured to perform digital beamforming in a second direction which is perpendicular to the first direction.

The beamforming apparatus may further include a controller configured to control components to calculate a delay profile for any one from among the plurality of transducer sub-blocks included in a corresponding transducer block, to calculate a delay time for each transducer included in the corresponding transducer sub-block based on the calculated delay profile, and to delay signals to be transmitted by the plurality of transducers included in the corresponding transducer sub-block and signals received from the plurality of transducers included in the corresponding transducer sub-block based on the calculated delay times.

The controller may be further configured to control the components to apply a same first delay time for digital beamforming to transducers which are arranged in a first direction in which the analog beamforming is performed, and to apply a same second delay time for analog beamforming to transducers which are arranged in a second direction in which the digital beamforming is performed.

The analog beamformer may be further configured to add analog-beamformed signals for the respective transducer blocks with respect to the received signals.

The analog beamformer may include a plurality of beamforming modules which correspond to the respective transducer blocks, each of the plurality of beamforming modules being configured to perform analog beamforming on signals which are transceived by the plurality of transducers included in the corresponding transducer sub-block based on a control signal received from the controller.

Each of the plurality of beamforming modules may include a signal delayer configured to delay signals to be transmitted by the plurality of transducers included in the corresponding transducer sub-block and signals received by the plurality of transducers included in the corresponding transducer sub-block, a switcher configured to perform a switching operation based on whether signals which are input into the signal delayer are the signals to be transmitted or the received signals, and an adder configured to add delayed signals which are output from the signal delayer when the signals which are input into the signal delayer are the received signals.

The analog beamformer may further include an analog adder configured to add signals which are output from the respective adders included in the plurality of beamforming modules.

In accordance with another aspect of one or more exemplary embodiments, a beamforming method for beamforming signals which are transmitted or received by a probe which includes a plurality of transducer blocks, each transducer block including a respective plurality of transducers, includes calculating a delay time for each of the plurality of transducers included in a corresponding one of the plurality of transducer blocks based on a delay profile which relates to the corresponding one of the plurality of transducer blocks, and performing analog beamforming on signals transceived by the plurality of transducers included in the corresponding transducer block based on the calculated delay times.

The performing the analog beamforming may include controlling a respective amount of delay for each of transmitted signals and received signals based on the calculated delay times.

The beamforming method may further include adding signals resulting from the performing the analog beamforming with respect to the received signals.

Each of the plurality of transducer blocks may include transducers that are one-dimensionally (1 D) arranged or two-dimensionally (2D) arranged.

The beamforming method may further include calculating the delay profile for each of the plurality of transducer blocks.

In accordance with another aspect, there is provided a beamforming apparatus for beamforming signals which are transmitted or received by a probe which comprises a plurality of transducers, the beamforming apparatus comprising a controller configured to control components to delay first signals to be transmitted by the plurality of transducers and second signals received by the plurality of transducers; and an analog beamformer which comprises a plurality of beamforming modules configured to perform first analog beamforming on the first signals and to peform second analog beamforming on the second signals based on a control signal which is received from the controller.

The plurality of transducers may be partitioned into transducer blocks, and the analog beamformer may be further configured to perform a respective first analog beamforming operation for each of the transducer blocks, and to perform a respective second analog beamforming operation for each of the transducer blocks.

The controller may be further configured to control the components to calculate a delay time for each of the plurality of transducers and to delay the first signals and the second signals based on the calculated delay times.

Each of the plurality of beamforming modules may comprise: a signal delayer configured to delay the first signals and to delay the second signals; a switcher configured to perform a switching operation based on whether signals which are input into the signal delayer are the first signals or the second signals; and an adder configured to add delayed signals which are output from the signal delayer when the signals which are input into the signal delayer are the second signals.

The signal delayer may comprises a plurality of delay lines configured to implement respective delay times based on the control signal.

In accordance with another aspect, there is provided a beamforming method for beamforming signals which are transmitted or received by a probe which comprises a plurality of transducers, the method comprising: calculating, for each of the plurality of transducers, a respective delay time; and performing, based on the calculated delay times, first analog beamforming on first signals to be transmitted by the plurality of transducers; and performing, based on the calculated delay times, second analog beamforming on second signals which are received by the plurality of transducers.

The plurality of transducers may partitioned into transducer blocks, and the performing the first analog beamforming may comprise performing a respective first analog beamforming operation for each of the transducer blocks, and the performing the second analog beamforming may comprise performing a respective second analog beamforming operation for each of the transducer blocks.

The performing the first analog beamforming may comprise controlling a respective amount of delay for each of the first signals based on the calculated delay times, and the performing the second analog beamforming may comprise controlling a respective amount of delay for each of the second signals based on the calculated delay times.

The method may further comprise adding signals resulting from the performing the second analog beamforming.

### BRIEF DESCRIPTION OF THE DRAWINGS

These and/or other aspects will become apparent and more readily appreciated from the following description of exemplary embodiments, taken in conjunction with the accompanying drawings of which:
FIG. 1 is a diagram which illustrates a concept of reception beamforming using a one-dimensional transducer;
FIG. 2A is a diagram which illustrates an example of a delay profile and a dynamic delay range (DDR), which are applied to beamforming without steering, and FIG. 2B is a diagram which illustrates an example of a delay profile and DDR, which are applied to beamforming with steering;
FIG. 3A is a diagram which illustrates a beamforming method and a DDR before an array transducer is blocked, FIG. 3B is a diagram which illustrates a beamforming method and a DDR for each respective block when an array transducer is divided into two blocks, and FIG. 3C is a diagram which illustrates a beamforming method and a DDR for each respective block when an array transducer is divided into four blocks;
FIG. 4 is a control block diagram of a beamforming apparatus, according to an exemplary embodiment;
FIG. 5 is a diagram which illustrates an analog beamformer illustrated in FIG. 4 in more detail;
FIG. 6 is a diagram which illustrates a schematic structure of a charge-coupled device (CCD) used as an analog delay line;
FIG. 7 is a diagram which illustrates an exemplary transmission beamforming operation performed by a beamforming apparatus, according to an exemplary embodiment;
FIG. 8 is a diagram which illustrates an exemplary reception beamforming operation performed by a beamforming apparatus, according to an exemplary embodiment;
FIG. 9 illustrates a control block of a beamforming apparatus, according to another exemplary embodiment;
FIG. 10 is a diagram which illustrates an exemplary transmission beamforming operation performed by a beamforming apparatus, according to another exemplary embodiment; and
FIG. 11 is a diagram which illustrates an exemplary reception beamforming operation performed by a beamforming apparatus, according to another exemplary embodiment.

### DETAILED DESCRIPTION

Reference will now be made in detail to exemplary embodiments, examples of which are illustrated in the accompanying drawings.

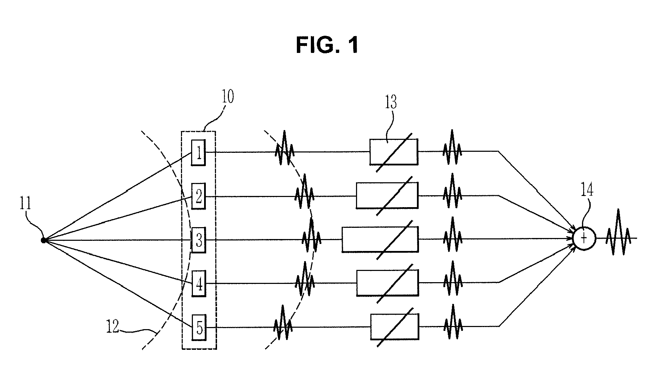

FIG. 1 is a diagram which illustrates a concept of reception beamforming using a one-dimensional transducer 10.

As illustrated in FIG. 1, five transducers are linearly arranged in the one-dimensional transducer array 10. A focal point 11 is a point at which source signals generated by the five transducers are focused in order to detect image information which relates to a two-dimensional (2D) image and which corresponds to a point inside an object (e.g., a human body, an animal body, etc.), in correspondence with a pixel location of the 2D image. The source signals received from the one-dimensional transducer array 10 are superposed at the focal point 11 via transmission beamforming. The superposed source signals are reflected from the focal point 11 and then retransmitted to the one-dimensional transducer array 10.

Each transducer of the one-dimensional transducer array 10 converts the reflected signal transmitted to each transducer into an electrical signal. These converted electrical signals have very low intensity, and thus, each electrical signal may not be analyzed individually. Thus, the electrical signals should be combined into one signal and should be analyzed as one combined signal. However, due to a distance difference between the transducers and the focal point 11, times taken for the reflected source signals to propagate to the transducers are different, and thus times taken to generate electrical signals by the transducers will correspondingly vary. Thus, in order to combine electrical signals output from the transducers as one signal, an electrical signal output from each respective transducer is delayed by as much as a time interval which varies inversely with respect to a corresponding distance between each respective transducer and the focal point 11, and then electrical signals from all the transducers of the one-dimensional transducer array 10 are combined at a point when the electrical signals are completely output from all the transducers of the one-dimensional transducer array 10.

In this aspect, as illustrated in FIG. 1, reception beamforming is performed by focusing received signals while variably delaying time by using a delay profile 12 that is calculated or stored based on a focus position (i.e., depth or distance) by a time delay unit (also referred to herein as a "time delay component" and/or as a "time delayer") 13, and then adding the received signals by an adder 14. In particular, an electrical signal output from a transducer among the transducers of the one-dimensional transducer array 10, which is furthest from the focal point 11, is lastly input to an ultrasonic volume scan apparatus. Thus, this electrical signal may be combined with electrical signals output from the other transducers without time delay. Combinations of electrical signals of the one-dimensional transducer array 10 in consideration of electrical signals output from the transducers and a difference in times taken to generate the electrical signals are referred to as reception beamforming.

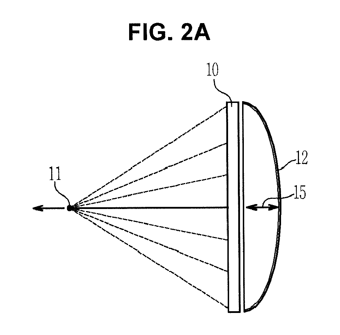

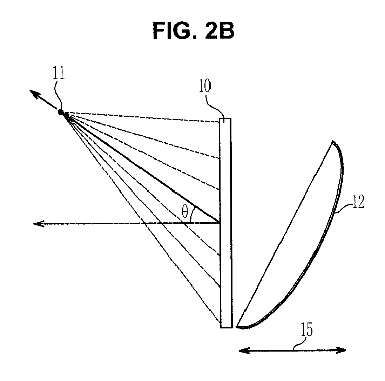

FIG. 2A is a diagram which illustrates an example of a delay profile 12 and a dynamic delay range (DDR) 15, which are applied to beamforming without steering. FIG. 2B is a diagram which illustrates an example of a delay profile 12 and a DDR 15, which are applied to beamforming with steering.

A scanning method for generating an ultrasonic image includes a method in which steering is not performed (refer to FIG. 2A) and a method in which steering is performed (refer to FIG. 2B, a steering angle θ). Comparing the delay profile 12 and the DDR 15 for beamforming as shown in FIG. 2A with the delay profile 12 and the DDR 15 for beamforming as shown in FIG. 2B, it may be seen that the DDR 15 is further increased when steering is performed. As a DDR increases, a time interval between a minimum delay time and a maximum delay time, which is to be covered by a beamformer, is increased. A DDR is determined based on a delay profile which may be calculated based on a focus position. In this regard, as the DDR increases, a size of delay line is increased so s to act as a limit in reducing a size of an analog beamformer.

Thus, in order to reduce the size of a DDR, a plurality of transducers included in an array transducer may be divided into a plurality of blocks, and a DDR may be calculated for each respective block based on a delay profile which corresponds to each block in order to reduce the DDR of the array transducer. In detail, beamforming is performed by dividing the plurality of transducers included in the array transducer into the plurality of blocks, applying a minimum delay time to a maximum delay time to only a delay profile of each block, adding time-delayed signals of transducers (channels) in each block, and then, collecting and re-combining the combined signals from the other blocks.

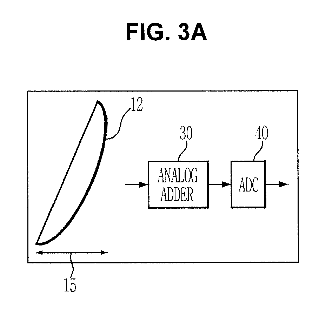

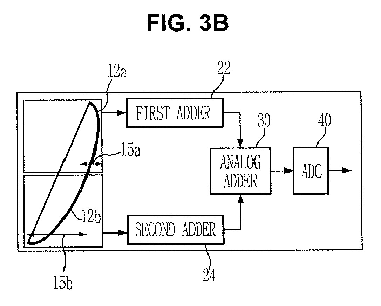

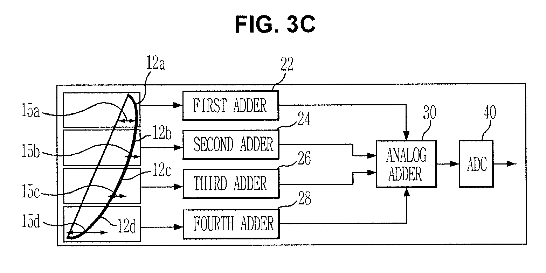

FIG. 3A is a diagram which illustrates a beamforming method and a DDR before an array transducer is blocked. FIG. 3B is a diagram which illustrates a beamforming method and a DDR for each respective block when an array transducer is divided into two blocks. FIG. 3C is a diagram which illustrates a beamforming method and a DDR for each respective block when an array transducer is divided into four blocks.

In FIGS. 3A, 3B, and 3C, it is assumed that the array transducer includes a total of 64 transducers. In FIG. 3A, since the array transducer is not blocked, the delay profile 12 is calculated with respect to all 64 transducers in order to acquire the DDR 15. Then, with regard to the 64 transducers, time-delayed signals of channels are combined by an analog adder 30 based on the calculated delay profile 12, and the resulting combined signals are transmitted to an analog to digital converter (ADC) 40.

FIG. 3B illustrates a case in which the 64 transducers included in the array transducer are divided into two blocks. Assuming that the 64 transducers are divided into the same number of blocks, a first delay profile 12a is calculated with respect to a first block which includes 32 transducers in order to acquire a first DDR 15a. In addition, a delay profile 12b of a second block which includes the other 32 transducers is calculated in order to acquire a second DDR 15b. Then, time-delayed signals of each transducer in the first block are combined by a first adder 22, time-delayed signals of each transducer in the second block are combined by a second adder 24, and then, the signals combined by the first adder 22 and the signals combined by the second adder 24 are re-combined by the analog adder 30 and transmitted to the ADC 40. Comparing the DDR 15 illustrated in FIG. 3A and the DDR 15b (which is wider from the two blocks) of the second block illustrated in FIG. 3B, it may be seen that a DDR is reduced when a plurality of transducers included in an array transducer is divided into two blocks.

FIG. 3C illustrates a case in which the 64 transducers included in the array transducer are divided into four blocks. Assuming that the 64 transducers are divided into the same number of blocks, the first delay profile 12a is calculated with respect to a first block which includes 16 transducers in order to acquire the first DDR 15a. The second delay profile 12b of a second block which includes 16 transducers is calculated in order to acquire the second DDR 15b. A third delay profile 12c of a third block which includes 16 transducers is calculated to acquire a third DDR 15c. Lastly, a fourth delay profile 12d of a fourth block which includes 16 transducers is calculated in order to acquire a fourth DDR 15d.

Then, time-delayed signals of each transducer in the first block are combined by the first adder 22, time-delayed signals of each transducer in the second block are combined by the second adder 24, time-delayed signals of each transducer in the third block are combined by a third adder 26, time-delayed signals of each transducer in the fourth block are combined by a fourth adder 28, and then, the signals combined by the first adder 22, the second adder 24, the third adder 26, and the fourth adder 28 are re-combined by the analog adder 30 and transmitted to the ADC 40. Comparing the DDR 15 of FIG. 3A, the DDR 15b of the second block of FIG. 3B, and the DDR 15d (which is the widest among the four blocks) of the fourth block of FIG. 3C, it may be seen that a DDR is most greatly reduced when a plurality of transducers included in an array transducer is divided into four blocks.

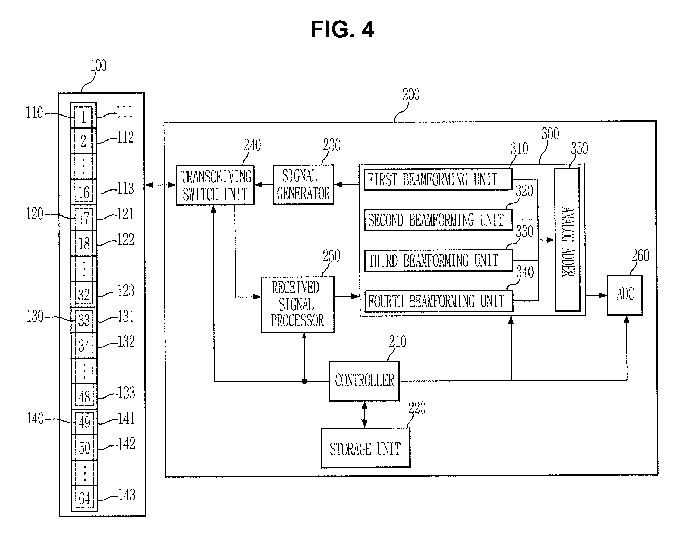

FIG. 4 is a control block diagram of a beamforming apparatus 200, according to an exemplary embodiment.

As illustrated in FIG. 4, an array transducer is installed in an ultrasonic probe 100 that transmits and receives an ultrasonic signal. In FIG. 4, the array transducer includes a total of 64 transducers that are one-dimensionally arranged, 16 transducers from a first transducer 111 and a second transducer 112 to a 16ₜₕ transducer 113 are set as a first transducer block 110, 16 transducers from a 17ₜₕ transducer 121 and an 18^{th} transducer 122 to a 32_{nd} transducer 123 are set as a second transducer block 120, 16 transducers from a 33^{rd} transducer 131 and a 34^{th} transducer 132 to a 48^{th} transducer 133 are set as a third transducer block 130, and 16 transducers from a 49^{th} transducer 141 and a 50^{th} transducer 142 to a 64^{th} transducer 143 are set as a fourth transducer block 140.

The beamforming apparatus 200 includes a controller 210, a storage unit (also referred to herein as a "storage device" and/or as a "storage") 220, a signal generator 230, a transceiving switch unit (also referred to herein as a "transceiving switcher") 240, a received signal processor 250, an analog beamformer 300, and an ADC 260.

The controller 210 controls the signal generator 230, the transceiving switch unit 240, the received signal processor 250, the analog beamformer 300, and the ADC 260 in order to control an overall operation of the beamforming apparatus 200. In more detail, the controller 210 may be configured control these components to calculate respective time delay values based on corresponding differences in distances between respective ones of the plurality of transducers 111 to 143 included in the ultrasonic probe 100 and a focal point of an object, and to form a transceiving beam based on the calculated time delay values in order to generate transceiving signals. When calculated time delay values are pre-stored and available, it may be possible to generate transceiving signals by using the pre-stored time delay values.

According to the exemplary embodiment illustrated in FIG. 4, the controller 210 may be configured control the aforementioned components to calculate delay profiles of the plurality of transducer blocks 110, 120, 130, and 140, to calculate respective delay times with respect to the transducers 111 to 113, 121 to 123, 131 to 133, and 141 to 143, which are respectively included in the transducer blocks 110, 120, 130, and 140, based on the delay profiles calculated for the respective transducer blocks, and to delay signals to be transmitted by the pluralities of transducers included in each of the transducer blocks 110, 120, 130, and 140 or signals received by the pluralities of transducers included in the transducer blocks 110, 120, 130, and 140 based on the calculated delay times. In particular, the controller 210 controls the aforementioned components to calculate a delay profile for each respective transducer block with respect to the plurality of transducer blocks 110, 120, 130, and 140 and to transmit and receive signals between the object and transducers of each block based on the delay profiles calculated for the respective blocks. A time delay value for analog beamforming may be calculated based on a corresponding distance between a transducer and a focal point, but the determination of the time delay value is not limited thereto.

In addition to the method for calculating a delay profile and delay time by the controller 210, a method for using a pre-stored delay profile and/or delay time may be applied. Delay profiles and/or delay times which correspond to the plurality of transducer blocks 110, 120, 130, and 140 may be pre-calculated based on a steering direction or a focal point and stored in the storage unit 220. In this case, the controller 210 may control beamforming by using the stored value.

The storage unit 220 stores the plurality of transducer blocks 110, 120, 130, and 140 set by dividing the plurality of transducers included in the array transducer. In particular, the storage unit 220 stores the number of the transducer blocks and combinations of the outputs of the transducers included in each of the transducer blocks 110, 120, 130, and 140. In this case, the plurality of transducers included in each of the transducer blocks 110, 120, 130, and 140 may be arranged to be adjacent to each other. As described above, when a pre-calculated delay profile and/or delay time is present, these values are stored in the storage unit 220.

The signal generator 230 generates a transmitted signal by using a transmission beam formed by the analog beamformer 300. The signal generator 230 may be, but is not limited to, an ultrasonic transmission pulser configured to generate a transmission pulse to be transmitted to an object via the ultrasonic probe 100.

The transceiving switch unit 240 performs a switching operation for transmitting and receiving signals in each of the transducers included in the transducer blocks 110, 120, 130, and 140 with respect to the ultrasonic transmission pulse generated by the signal generator 230 and/or the signals received by the ultrasonic probe 100.

The received signal processor 250 performs a predetermined processing operation on an ultrasonic echo signal which is received from the array transducer in the ultrasonic probe 100. For example, the received signal processor 250 may include a low noise amplifier (LNA) (not shown) which is configured to reduce noise with respect to an analog signal received from the array transducer and/or a variable gain amplifier (VGA) (not shown) which is configured to control a gain value based on an input signal. In this case, the VGA may be configured to perform, but is not limited to performing, time gain compensation (TGC) in order to compensate a gain based on a distance from a focal point.

The analog beamformer 300 forms a transmission beam based on a control signal generated by the controller 210, outputs the transmission beam to the signal generator 230, and synthesizes signals received by the transducers included in each of the transducer blocks 110, 120, 130, and 140 based on time delay values for analog beamforming in order to generate a plurality of analog signals with respect to the plurality of transducer blocks 110, 120, 130, and 140.

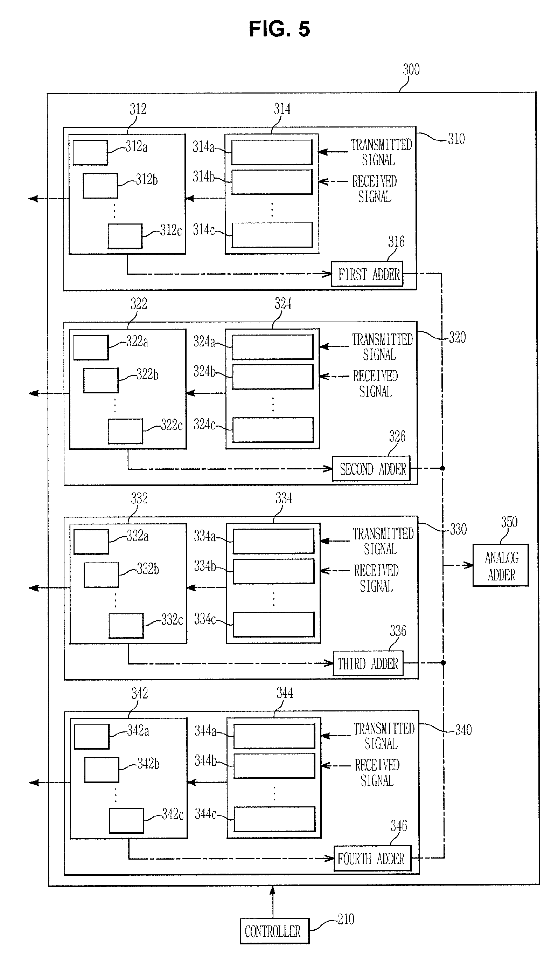

The analog beamformer 300 includes first, second, third, and fourth beamforming units (also referred to herein as "beamforming modules") 310, 320, 330, and 340 and an analog adder 350. Detailed components of the analog beamformer 300 will be described below in detail with reference to FIG. 5.

The ADC 260 digitizes each of the plurality of analog signals which is generated by the analog beamformer 300 into a digital signal. Thus, the ADC 260 generates a plurality of digital signals.

FIG. 5 is a diagram which illustrates the analog beamformer 300 illustrated in FIG. 4 in more detail.

As illustrated in FIG. 5, the analog beamformer 300 includes the first beamforming unit 310 which is configured to beamform a signal that is transmitted or received by a plurality of transducers 111 to 113 belonging to the first transducer block 110, the second beamforming unit 320 which is configured to beamform a signal that is transmitted or received by a plurality of transducers 121 to 123 belonging to the second transducer block 120, the third beamforming unit 330 which is configured to beamform a signal that is transmitted or received by the plurality of transducers 131 to 133 belonging to the third transducer block 130, the fourth beamforming unit 340 which is configured to beamform a signal that is transmitted or received by the plurality of transducers 141 to 143 belonging to the fourth transducer block 140, and the analog adder 350. Each of the first beamforming unit 310, the second beamforming unit 320, the third beamforming unit 330, and the fourth beamforming unit 340 has the same configuration, and thus only a configuration of the first beamforming unit 310 will be described in detail.

As illustrated in FIG. 5, the first beamforming unit 310 includes a first switch unit (also referred to herein as a "switcher" and/or as a "switch") 312, a first signal delay unit (also referred to herein as a "signal delayer") 314, and a first adder 316. In FIG. 5, an arrow indicated by a solid line is a transmitted signal, and an arrow indicated by a dashed dotted line is a received line. The first signal delay unit 314 delays a signal to be transmitted or received by the transducers 111 to 113 included in the first transducer block 110. For example, the first signal delay unit 314 receives the control signal generated by the controller 210 and delays signals input to the first signal delay unit 314 by as much as a delay time which is determined based on the control signal. In this case, the signals input to the first signal delay unit 314 may each be a pulse which corresponds to a signal to be transmitted from the controller 210 or an echo signal which is reflected from an object.

The first signal delay unit 314 may include a plurality of analog delay lines 314 and 314b to 314c for implementing transmission and reception delay times based on the control signal. In this case, delay times used for transmission and delay time used for reception in the transducers 111 to 113 are the same. Thus, the analog beamformer 300 may process transmission and reception in terms of analog.

The first switch unit 312 is provided at an output end of the first signal delay unit 314 and may be embodied by any one or more of various devices configured to perform a switching operation. Switching devices 312a and 312b to 312c included in the first switch unit 312 perform a switching operation based on whether a signal output from the first signal delay unit 314 is a signal to be transmitted to an object or a signal reflected from the object. For example, when the signal output from the first signal delay unit 314 is to be transmitted to the object, the switching devices 312a to 312c in the first switch unit 312 performs the switching operation so as to output the signal output from the first signal delay unit 314 to the signal generator 230. When the signal output from the first signal delay unit 314 is reflected from the object, the switching devices 312a to 312c in the first switch unit 312 perform the switching operation in order to output the signal output from the first signal delay unit 314 to the first adder 316.

Accordingly, the analog beamformer 300 may control a transmission operation and a reception operation with respect to transducers of each of the transducer blocks 110, 120, 130, and 140 by using switching devices included in the switching units 312, 322, 332, and 342 provided at an output end of each of the signal delay units 314, 324, 334, and 344.

During a reception beamforming operation, received signals of the transducers 111 to 113 belonging to the first transducer block 110 are combined by the first adder 316, received signals of the transducers 121 to 123 belonging to the second transducer block 120 are combined by a second adder 326, received signals of the transducers 131 to 133 belonging to the third transducer block 130 are combined by a third adder 336, and received signals of the transducers 141 to 143 belonging to the fourth transducer 140 are combined by a fourth adder 346. In addition, signals output from each of the adders 316, 326, 336, and 346 are combined by the analog adder 350.

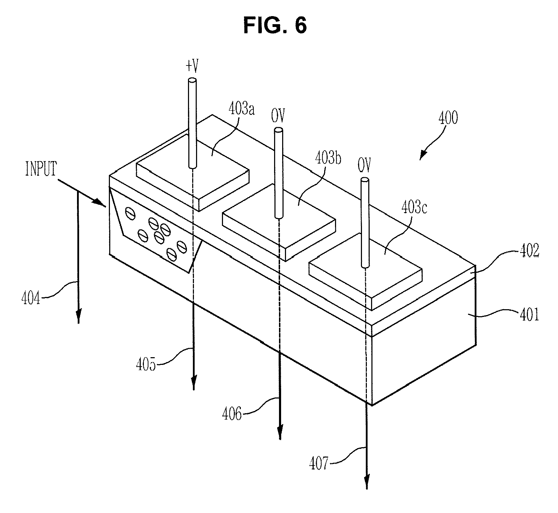

FIG. 6 is a diagram which illustrates a schematic structure of a charge-coupled device (CCD) 400 which is used as an analog delay line. Analog signal lines 314a to 344c included in the first to fourth signal delay units 314, 324, 334, and 344 described with reference to FIG. 5 may be used as the CCD 400.

The CCD 400 is a semiconductor integrated circuit device which uses accumulation and movement of electric charges and which is includes an insulating layer 402 formed to a thickness of about 0.1 mm on a semiconductor surface 401 and metallic electrodes 403a, 403b, and 403c which are arranged on the insulating layer 402. The CCD 400 is a device that controls voltages of the metallic electrodes 403a, 403b, and 403c in order to move electric charges to a portion of the semiconductor surface 401 which has a low voltage, and to sequentially transmit accumulating electric charges. In one aspect, the insulating layer 402 may be formed of silicon dioxide (SiO₂).

When a signal is input to the CCD 400, the CCD 400 outputs a signal without delay, such as a first output 404. A voltage may be repeatedly applied to and removed from the metallic electrodes 403a, 403b, and 403c. In this regard, when a voltage is applied to the metallic electrodes 403a, 403b, and 403c, electric charges accumulate below a portion of the insulating layer 402, which portion is positioned just below a voltage-applied electrode among the metallic electrodes 403a, 403b, and 403c, according to Coulomb's law. As illustrated in FIG. 6, when a voltage is applied to the metallic electrode 403a, input electric charges accumulate under a portion of the insulating layer 402, which portion is positioned just below the metallic electrode 403a. While the electric charges accumulate below the metallic electrode 403a, if the voltage is removed from the metallic electrode 403a and simultaneously a voltage is applied to the metallic electrode 403b, the electric charges are moved below a portion of the insulating layer 402, which portion is positioned just below the metallic electrode 403b. Under the same principle, when the voltage is removed from the metallic electrode 403b and a voltage is applied to the metallic electrode 403c, the electric charges are moved below a portion of the insulating layer 402, which portion is positioned just below the metallic electrode 403c. When the voltage application is controlled at regular time intervals, predetermined time delayed outputs 405, 406, and 407 may be acquired. As illustrated in FIG. 6, when a CCD is used as an analog delay line, an output may be delayed by as much as the number of the arranged metallic electrodes 403a, 403b, and 403c, and thus, a total of four outputs from three delayed outputs and one undelayed output may be obtained in the example illustrated in FIG. 6.

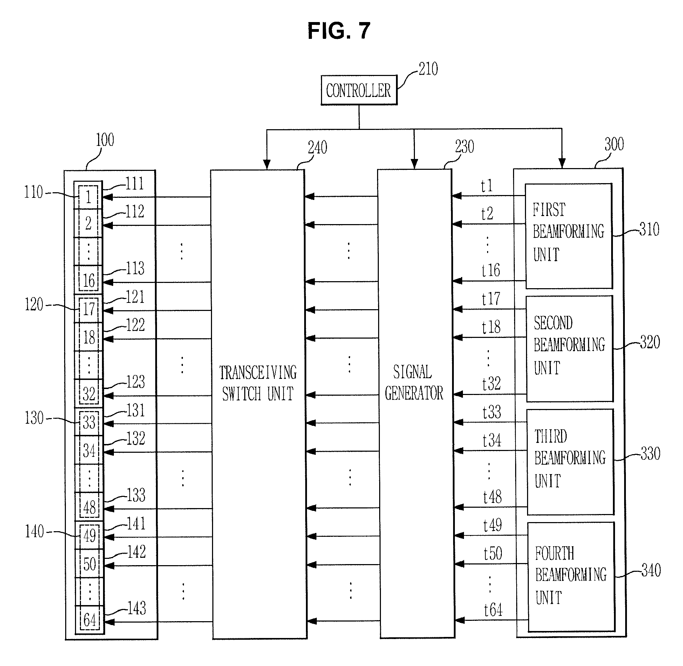

FIG. 7 is a diagram which illustrates an exemplary transmission beamforming operation performed by a beamforming apparatus, according to an exemplary embodiment.

Referring to FIG. 7, the ultrasonic probe 100 includes a total of four transducer blocks 110, 120, 130, and 140. FIG. 7 illustrates sixteen transducers 111 to 113, sixteen transducers 121 to 123, sixteen transducers 131 to 133, and sixteen transducers 141 to 143, respectively included in the transducer blocks 110, 120, 130, and 140.

The controller 210 may calculate respective delay profiles of the plurality of transducer blocks 110, 120, 130, and 140 or use pre-stored delay profiles. The controller 210 controls components to calculate delay times for analog beamforming of each of the transducers 111 to 113, 121 to 123, 131 to 133, and 141 to 143, respectively included in the transducer blocks 110, 120, 130, and 140, based on delay profiles, calculated or stored for respective transducer blocks, and to transmit signals to the object from the transducers 111 to 113, 121 to 123, 131 to 133, and 141 to 143 based on the calculated time delay values. When a delay time which corresponds to a delay profile is pre-stored, the delay time may be used without calculation.

The transducers 111 to 113 included in the first transducer block 110 will now be described in more detail. The transducer 111 transmits a signal, to which a time delay value t1 for analog beamforming is applied, to the object, the transducer 112 transmits a signal, to which a time delay value t2 for analog beamforming is applied, to the object, and the transducer 113 transmits a signal, to which a time delay value t16 for analog beamforming is applied, to the object.

The transducers 121 to 123 included in the second transducer block 120 will now be described. The transducer 121 transmits a signal, to which a time delay value t17 for analog beamforming is applied, to the object. In a similar manner, transducers included in the second transducer block 120 to fourth transducer block 140 may also transmit signals to which corresponding time delay values are applied, to the object.

The signal generator 230 generates an electrical signal based on a transmission beam formed by the analog beamformer 300, and the transceiving switch unit 240 is switched to transmit signals to the transducers 111 to 143.

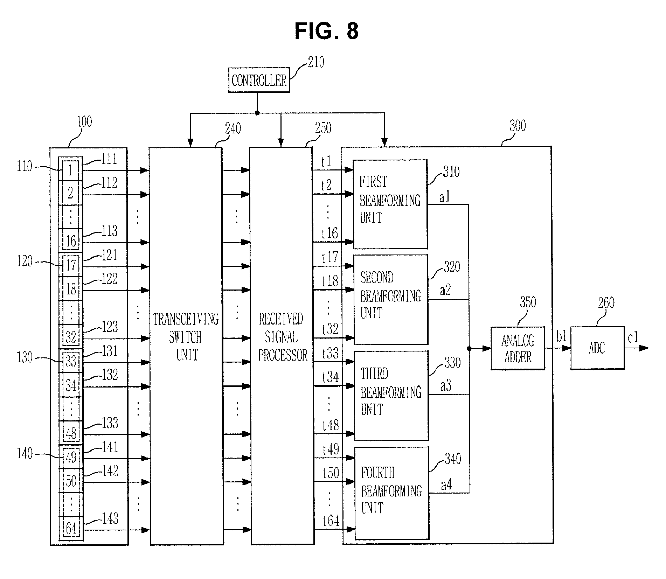

FIG. 8 is a diagram which illustrates an exemplary reception beamforming operation performed by a beamforming apparatus, according to an exemplary embodiment.

Referring to FIG. 8, the ultrasonic probe 100 includes total of four transducer blocks 110, 120, 130, and 140. FIG. 8 illustrates sixteen transducers 111 to 113, sixteen transducers 121 to 123, sixteen transducers 131 to 133, and sixteen transducers 141 to 143, respectively included in transducer blocks.

The transceiving switch unit 240 is switched to enable signals to be respectively received by the corresponding transducers 111, 112,...143. The received signal processor 250 performs a predetermined process, such as noise reduction and/or gain amplification, on the received signals.

The first beamforming unit 310 synthesizes signals that are received by the transducers 111 to 113 included in the first transducer block 110 and processed by the received signal processor 250 based on delay time values t1 to t16 for analog beamforming, used to form a transmission beam, in order to generate an analog signal a1. In the same manner, the first, second, third, and fourth beamforming units 310 to 340 synthesize signals received by respective transducers included in each of the transducer blocks 110, 120, 130, and 140 in order to generate four analog signals a1, a2, a3, and a4.

The analog adder 350 combines the signals a1, a2, a3, and a4 respectively output from the first, second, third, and fourth beamforming units 310 to 340 in order to generate one analog signal b1.

The ADC 260 converts one analog signal b1 into one digital signal c1.

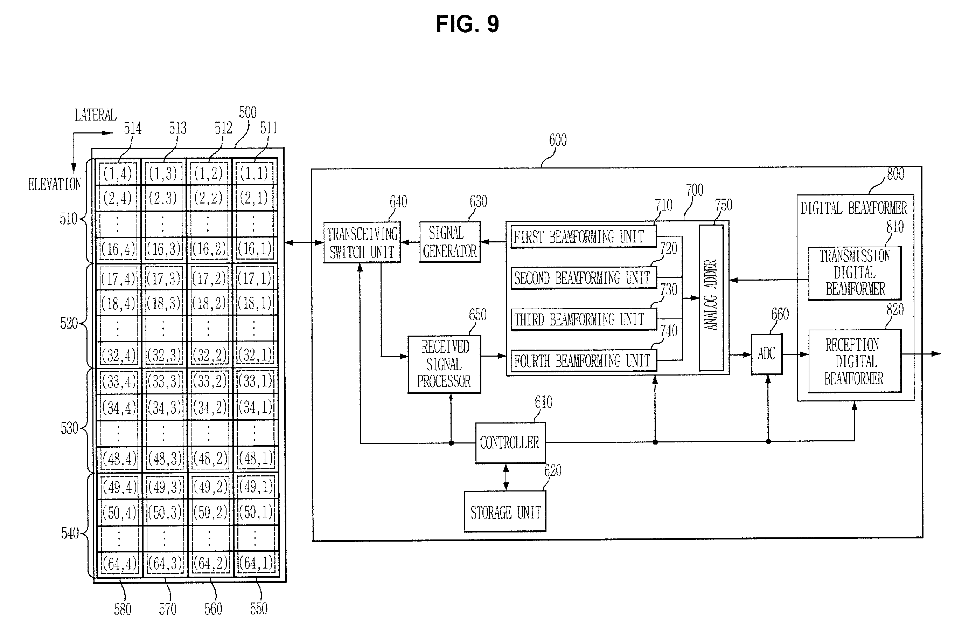

FIG. 9 illustrates a control block of a beamforming apparatus 600, according to another exemplary embodiment.

As illustrated in FIG. 9, an array transducer is installed in an ultrasonic probe 500 that that transmits and receives an ultrasonic signal. In FIG. 9, the array transducer includes a total of 256 (64 x4=256) transducers that are two-dimensionally arranged, of which 64 transducers (1,1) to (16,4) arranged from a first row to a 16^{th} row are set as a first transducer block 510, 64 transducers (17,1) to (32,4) arranged from a 17^{th} row to a 32^{nd} row are set as a second transducer block 520, 64 transducers (33,1) to (48,4) arranged from a 33^{rd} row to a 48^{th} row are set as a third transducer block 530, and 64 transducers (49,1) to (64,4) arranged from a 49^{th} row to a 64^{th} row are set as a fourth transducer block 540.

In the exemplary embodiment illustrated in FIG. 9, each of the transducer blocks 510, 520, 530, and 540 includes a plurality of transducer sub-blocks. For example, the first transducer block 510, which includes the 64 transducers (1,1) to (16,4), may include a first transducer sub-block 511 which includes 16 transducers (1,1) to (16,1), a second transducer sub-block 512 which includes 16 transducers (1,2) to (16,2), a third transducer sub-block 513 which includes 16 transducers (1,3) to (16,3), and a fourth transducer sub-block 514 which includes 16 transducers (1,4) to (16,4).

According to the exemplary embodiment illustrated in FIG. 9, an array transducer which includes one-line transducers arranged in a vertical (elevation) direction is set as one transducer sub-array. In this aspect, 64 transducers (1,1) to (64,1) arranged in a first right column are set as a first transducer sub-array 550, 64 transducers (1,2) to (64,2) arranged in a second right column are set as a second transducer array sub-560, 64 transducers (1,3) to (64,3) arranged in a third right column are set as a third transducer sub-array 570, and 64 transducers (1,4) to (64,4) arranged in a fourth right column are set as a fourth transducer sub-array 580.

The beamforming apparatus 600 includes a controller 610, a storage unit 620, a signal generator 630, a transceiving switch unit 640, a received signal processor 650, an analog beamformer 700, an ADC 660, and a digital beamformer 800. The analog beamformer 700 includes first, second, third, and fourth beamforming units 710, 720, 730, and 740, and an analog adder 750. The digital beamformer 800 includes a transmission digital beamformer 810 and a reception digital beamformer 820.

The controller 610 controls the signal generator 630, the transceiving switch unit 640, the received signal processor 650, the analog beamformer 700, the ADC 660, and the digital beamformer 800 in order to control an overall operation of the beamforming apparatus 600. In more detail, the controller 610 may control these components to calculate time delay values based on respective differences in distances between a plurality of transducers included in the ultrasonic probe 500 and a focal point of an object, and to form a transceiving beam based on the calculated time delay values in order to generate transceiving signals. When calculated time delay values are pre-stored in the storage unit 620 and available, the controller 610 may control these components to generate transceiving signals by using the pre-stored time delay values.

The controller 610 controls these components to calculate time delay values for digital beamforming with respect to the transducer sub-arrays 550, 560, 570, and 580 in order to transmit and receive signals between the transducers and the object based on the calculated time delay values. In addition, the controller 610 controls these components to calculate time delay values for analog beamforming with respect to a transducer sub-block of any one of the plurality of transducer blocks 510, 520, 530, and 540 and to transmit and receive signals to and from the object. In this case, among transducers included in the plurality of transducer blocks 510, 520, 530, and 540, transducers positioned at corresponding positions in a first direction (e.g., a horizontal direction or a lateral direction) which is perpendicular to a second direction (e.g., a vertical direction or an elevation direction) in which the transducer sub-arrays 550, 560, 570, and 580 are arranged, or a third direction in which transducer sub-blocks are arranged, have the same time delay value for analog beamforming. Thus, the controller 610 calculates time delay values for analog beamforming with respect to only transducers included in any one of the plurality of transducer sub-arrays included in each of the transducer blocks 510, 520, 530, and 540. A time delay value for analog beamforming may be calculated based on a distance between a transducer and a focal point, but a determination of a time delay value is not limited thereto.

In order to calculate a time delay values for analog beamforming, the controller 610 calculates a delay profile of any one of the plurality of transducer sub-blocks included in each of the transducer blocks 510, 520, 530, and 540, and calculates respective delay times (time delay values) of corresponding transducers included in each transducer sub-block based on the calculated delay profile. For example, in order to respectively calculate time delay values for analog beamforming with respect to 64 transducers included in the first transducer block 510 among the plurality of transducer blocks 510, 520, 530, and 540, the controller 610 calculates a delay profile with respect to the first transducer sub-block 511 from among the plurality of transducer sub-blocks 511, 512, 513, and 514 included in the first transducer block 510, and calculates delay times with respect to each of the transducers (1,1), (2,1)...(16,1) included in the first transducer sub-block 511 based on the calculated delay profile. As described above, transducers positioned at corresponding positions in a first direction (e.g., a horizontal direction or a lateral direction) which is perpendicular to a second direction (e.g., a vertical direction or an elevation direction) in which the transducer sub-blocks 511, 512, 513, and 514 are arranged have the same time delay value for analog beamforming. Thus, delay times calculated with respect to the transducers (1,1), (2,1), ...(16,1) included in the first transducer sub-block 511 may be applied to transducers (1,2), (2,2), ...(16,2) included in the second transducer sub-block 512, transducers (1,3), (2,3), ...(16,3) included in the third transducer sub-block 513, and transducers (1,4), (2,4), ...(16,4) included in the fourth transducer sub-block 514, in the same way.

As described above, when a pre-stored time delay value is present, the controller 610 may use the pre-stored time delay value without calculation of a time delay value.

The controller 610 controls the aforementioned components to delay signals to be transmitted from a plurality of transducers included in each of the transducer blocks 510, 520, 530, and 540 and signals received from a plurality of transducers included in each of the transducer blocks 510, 520, 530, and 540 based on the calculated time delay values (i.e., time delay values for digital beamforming and time delay values for analog beamforming).

The controller 610 controls the analog beamformer 700 to perform analog beamforming in a first direction (e.g., a vertical direction or an elevation direction) in which the transducer sub-arrays 550, 560, 570, and 580 are arranged or a second direction in which the transducer sub-blocks 511, 512, 513, and 514 are arranged, or controls the digital beamformer 800 to perform digital beamforming in a first direction (e.g., a horizontal direction or a lateral direction) which is perpendicular to a second direction in which the transducer sub-blocks 511, 512, 513, and 514 or the sub-transducer arrays 550, 560, 570, and 580 are arranged.

The storage unit 620 stores the plurality of transducer blocks 510, 520, 530, and 540 set by dividing the plurality of transducers included in the array transducer, the plurality of transducer sub-blocks set by dividing each of the transducer blocks 510, 520, 530, and 540, and the plurality of transducer sub-arrays 550, 560, 570, and 580 in which an array transducer including one-line transducers arranged in a vertical direction (an elevation direction) is set as one transducer sub-array. In this case, the transducer blocks 510, 520, 530, and 540 are configured in such a way that the plurality of transducers included in each of the transducer blocks 510, 520, 530, and 540 are arranged adjacent to each other. As described above, when a pre-calculated delay profile and/or delay time is present, these values are stored in the storage unit 620.

The signal generator 630 generates a transmitted signal by using a transmission beam formed by the analog beamformer 700. The signal generator 630 may be, but is not limited to, an ultrasonic transmission pulser configured to generate a transmission pulse to be transmitted to an object via the ultrasonic probe 500.

The transceiving switch unit 640 performs a switching operation for transmitting and receiving signals in each of the transducers included in the transducer sub-arrays 550, 560, 570, and 580 with respect to at least one of the signal (the ultrasonic transmission pulse) generated by the signal generator 630 and the signal received by the ultrasonic probe 500.

The received signal processor 650 performs a predetermined processing operation on an ultrasonic echo signal received from the array transducer in the ultrasonic probe 500. For example, the received signal processor 650 may include a low noise amplifier (LNA) (not shown) configured for reducing noise with respect to an analog signal received from the array transducer and/or a variable gain amplifier (VGA) (not shown) configured to control a gain value based on an input signal. In this case, the VGA may be configured for performing, but is not limited to performing, time gain compensation (TGC) to compensate a gain based on a distance from a focal point.

The analog beamformer 700 forms a transmission beam based on a control signal received from the controller 610, outputs the transmission beam to the signal generator 630, and synthesizes signals received by respective transducers included in the transducer blocks 510, 520, 530, and 540 in order to generate a plurality of analog signals with respect to the plurality of transducer blocks.

For example, the analog beamformer 700 performs analog beamforming based on a respective time delay value for analog beamforming based on a distance from a focal point for each transducer included in any one (e.g., a first transducer sub-block) of the plurality of transducer sub-blocks included in each of the transducer blocks 510, 520, 530, and 540. In this case, among transducers included in each of the plurality of transducer blocks 510, 520, 530, and 540, transducers (e.g., four transducers (1,1) to (1,4) arranged in a first row) positioned at corresponding positions in a first direction (e.g., a horizontal direction or a lateral direction) which is perpendicular to a second direction in which transducer sub-blocks (e.g., 511 to 514) are arranged have the same time delay value for analog beamforming.

In this case, any one of the transducer blocks may be, but is not limited to, a transducer block closest to the focal point. In this aspect, various cases may be considered, for example, any one of the transducer blocks may be a transducer block furthest from the focal point, an intermediate transducer block of the transducer blocks, or the like.

The ADC 660 converts a plurality of analog signals generated by the analog beamformer 700 into digital signals. Thus, the ADC 660 generates a plurality of digital signals and outputs the generated digital signals to the digital beamformer 800.

The digital beamformer 800 forms a transmission beam based on the control signal received from the controller 610 and outputs the transmission beam to the analog beamformer 700 (transmission digital beamforming), and synthesizes the plurality of digital signals converted by the ADC 660 based on the time delay values calculated by the transducer sub-arrays 550, 560, 570, and 580 (reception digital beamforming).

For example, the transmission digital beamformer 810 forms a transmission beam based on the control signal received from the controller 610 and outputs the transmission beam to the analog beamformer 700, and the reception digital beamformer 820 synthesizes the plurality of digital signals converted by the ADC 660 based on the time delay values for digital beamforming, calculated by the transducer sub-arrays 550, 560, 570, and 580, in order to form a reception beam.

During reception beamforming, the analog beamformer 700 synthesizes signals received by the transducers included in the transducer blocks 510, 520, 530, and 540 based on time delay values for analog beamforming in order to generate a plurality of analog signals with respect to the plurality of transducer blocks 510, 520, 530, and 540, the ADC 660 converts the plurality of analog signals generated by the analog beamformer 700 into digital signals, and the reception digital beamformer 820 synthesizes the plurality of digital signals converted by the ADC 660 based on the time delay values for digital beamforming, calculated by the controller 610.

Likewise, the digital beamformer 800 performs dynamic focusing for forming a transceiving beam in consideration of the time delay values for digital beamforming, calculated by the transducer sub-arrays 550, 560, 570, and 580. For example, the reception digital beamformer 820 performs dynamic focusing on the plurality of analog signals generated by the analog beamformer 700, thereby dramatically reducing the number of cables connected to the digital beamformer 800.

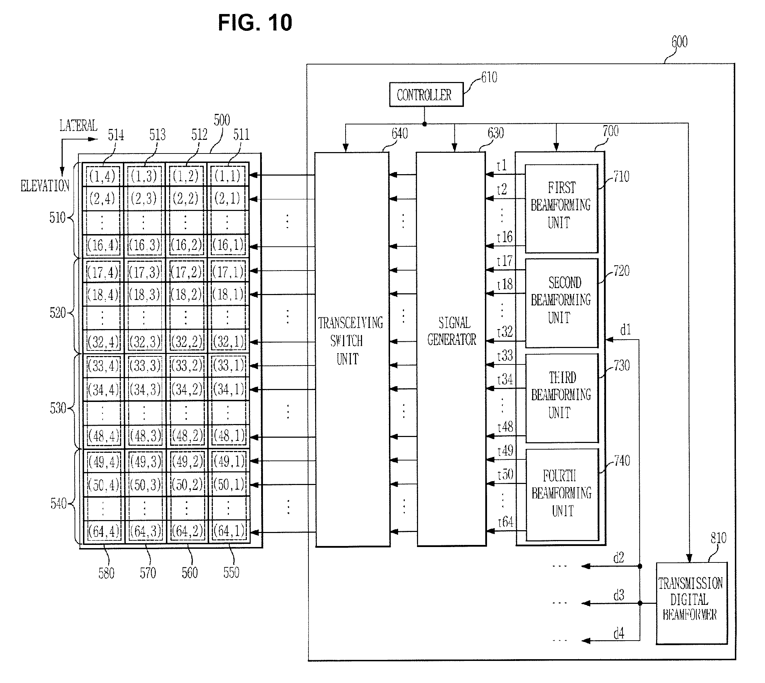

FIG. 10 is a diagram which illustrates an exemplary transmission beamforming operation performed by a beamforming apparatus, according to another exemplary embodiment.

As illustrated in FIG. 10, an array transducer is installed in the ultrasonic probe 500 that transmits and receives an ultrasonic signal. In the exemplary embodiment illustrated in FIG. 10, for convenience of description, an array transducer including all 16 rows of transducers arranged in a horizontal direction (i.e., a lateral direction) is set as one transducer block, one transducer block is re-set as a plurality of transducer sub-blocks, and one row of transducers arranged in a vertical direction (i.e., an elevation direction) are set as one transducer sub-array.

As illustrated in FIG. 10, the ultrasonic probe 500 includes four transducer blocks 510, 520, 530, and 540, each of which includes four sub-transducer blocks (i.e., regions indicated by dotted lines of FIG. 10).

The controller 610 controls components to calculate a time delay value for digital beamforming with respect to each of the transducer sub-arrays 550, 560, 570, and 580, to calculate a time delay value for analog beamforming with respect to each transducer included in any one of a plurality of transducer sub-blocks included in each of the transducer blocks 510, 520, 530, and 540, and to transmit signals to the object from the transducers (1,1) to (64,4) based on the calculated time delay values.

The transmission digital beamformer 810 applies time delay values d1, d2, d3, and d4 for digital beamforming to the transducers included in the transducer sub-arrays 550, 560, 570, and 580, and the analog beamformer 700 applies time delay values t1 to t64 for analog beamforming to the transducers (1,1) to (64,1), (1,2) to (64,2), (1,3) to (64,3), and (1,4) to (64,4) included in the transducer sub-arrays 550, 560, 570, and 580.

Hereinafter, for example, time delay values for analog beamforming, applied to each of the transducers (1,1), (1,2), ...(16,4) included in, for example, the first transducer block 510, will be described in more detail.

The transducer (1,1) belonging to the first transducer sub-block 511 in the first transducer block 510 transmits signals to the object based on time delay values to which both time delay value d1 for digital beamforming and time delay value t1 for analog beamforming are applied, the transducer (2,1) transmits signals to the object based on time delay values to which both time delay value d1 for digital beamforming and time delay value t2 for analog beamforming are applied, and the transducer (16,1) transmits signals to the object based on time delay values to which both time delay value d1 for digital beamforming and time delay value t16 for analog beamforming are applied, in the same manner.

The transducer (1,2) belonging to the second transducer sub-block 512 in the first transducer block 510 transmits signals to the object based on time delay values to which both time delay value d2 for digital beamforming and time delay value t1 for analog beamforming are applied, the transducer (2,2) transmits signals to the object based on time delay values to which both time delay value d2 for digital beamforming and time delay value t2 for analog beamforming are applied, and the transducer (16,2) transmits signals to the object based on time delay values to which both time delay value d2 for digital beamforming and time delay value t16 for analog beamforming are applied, in the same manner.

The transducer (1,3) belonging to the third transducer sub-block 513 in the first transducer block 510 transmits signals to the object based on time delay values to which both time delay value d3 for digital beamforming and time delay value t1 for analog beamforming are applied, the transducer (2,3) transmits signals to the object based on time delay values to which both time delay value d3 for digital beamforming and time delay value t2 for analog beamforming are applied, and the transducer (16,3) transmits signals to the object based on time delay values to which both time delay value d3 for digital beamforming and time delay value t16 for analog beamforming are applied, in the same manner.

In addition, the transducer (1,4) belonging to the fourth transducer sub-block 514 in the first transducer block 510 transmits signals to the object based on time delay values to which both time delay value d4 for digital beamforming and time delay value t1 for analog beamforming are applied, the transducer (2,4) transmits signals to the object based on time delay values to which both time delay value d4 for digital beamforming and time delay value t2 for analog beamforming are applied, and the transducer (16,4) transmits signals to the object based on time delay values to which both time delay value d4 for digital beamforming and time delay value t16 for analog beamforming are applied, in the same manner.

In this manner, time delay values with respect to other transducers included in the second transducer block 520, the third transducer block 530, and the fourth transducer block 540 may be obtained.

Likewise, the transducers (1,1), (1,2), (1,3), and (1,4), which are arranged at corresponding positions in a lateral direction which is perpendicular to a direction in which the transducer sub-blocks (e.g., 511, 512, 513, and 514) are arranged, have the same time delay value t1 for analog beamforming.

The signal generator 630 generates an electrical signal based on the transmission beam formed by the analog beamformer 700, and the transceiving switch unit 640 is switched in order to transmit signals to each of the transducer sub-arrays 550, 560, 570, and 580.

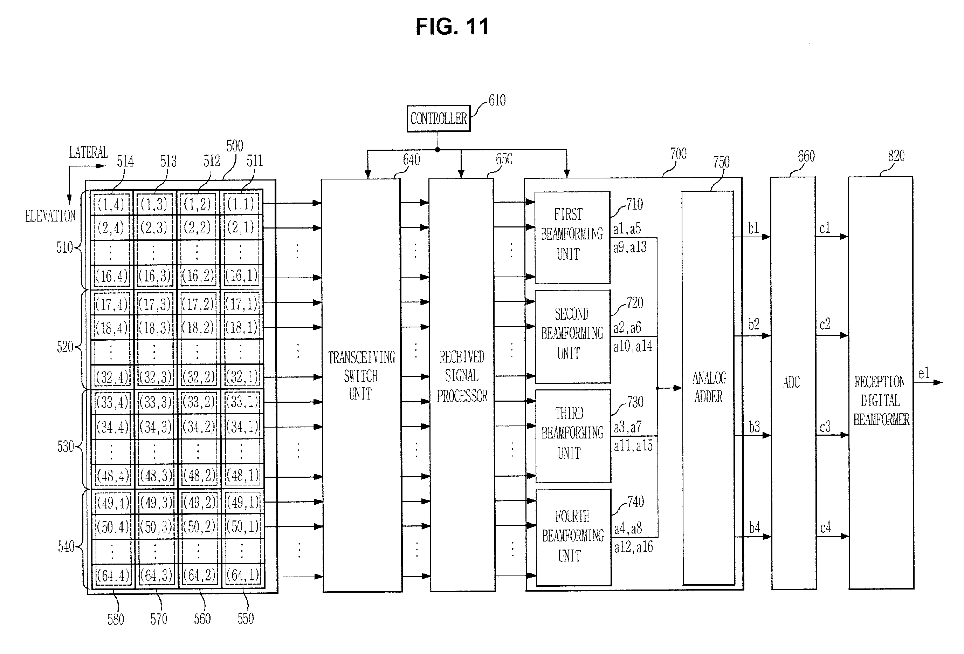

FIG. 11 is a diagram which illustrates an exemplary reception beamforming operation performed by a beamforming apparatus, according to another exemplary embodiment.

As illustrated in FIG. 11, an array transducer is installed in the ultrasonic probe 500 that transmits and receives an ultrasonic signal. In the exemplary embodiment illustrated in FIG. 11, for convenience of description, an array transducer which includes all 16 rows of transducers arranged in a horizontal direction (i.e., a lateral direction) is set as one transducer block, one transducer block is re-set as a plurality of transducer sub-blocks, and one row of transducers arranged in a vertical direction (i.e., an elevation direction) are set as one transducer sub-array.

As illustrated in FIG. 11, the ultrasonic probe 500 includes four transducer blocks 510, 520, 530, and 540, each of which includes four transducer sub-blocks (i.e., regions indicated by dotted lines of FIG. 11).

The transceiving switch unit 640 is switched to enable each of the transducer sub-blocks 550, 560, 570, and 580 to receive respective signals. The received signal processor 650 performs a predetermined process, such as noise reduction and/or gain amplification, on the received signals.

The first beamforming unit 710 in the analog beamformer 700 synthesizes signals which are received by 16 transducers (1,1) to (16,1) arranged in a first right column of the first transducer block 510 and processed by the received signal processor 650 based on respective delay signal values t1 to t16 for analog beamforming used for forming a transmission beam in order to generate an analog signal a1. In addition, the second beamforming unit 720 synthesizes signals which are received by 16 transducers (17,1) to (32,1) arranged in a first right column of the second transducer block 520 and processed by the received signal processor 650 based on respective delay signal values t17 to t32 for analog beamforming used for forming a transmission beam in order to generate an analog signal a2. In this manner, the first, second, third, and fourth beamforming units 710, 720, 730, 740 in the analog beamformer 700 synthesize signals which are received by the transducers (1,1) to (16,1), (17,1) to (32,1), (33,1) to (48,1), and (49,1) to (64,1) arranged in a first right column of each of the transducer blocks 510, 520, 530, and 540 in order to generate four analog signals a1, a2, a3, and a4, respectively.

The first beamforming unit 710 in the analog beamformer 700 synthesizes signals which are received by 16 transducers (1,2) to (16,2) arranged in a second right column of the first transducer block 510 and processed by the received signal processor 650 based on respective delay signal values t1 to t16 for analog beamforming used for forming a transmission beam in order to generate an analog signal a5. In addition, the second beamforming unit 720 synthesizes signals which are received by 16 transducers (17,2) to (32,2) arranged in a second right column of the second transducer block 520 and processed by the received signal processor 650 based on respective delay signal values t17 to t32 for analog beamforming used for forming a transmission beam in order to generate an analog signal a6. In this manner, the first, second, third, and fourth beamforming units 710, 720, 730, 740 in the analog beamformer 700 synthesize signals which are received by the transducers (1,2) to (16,2), (17,2) to (32,2), (33,2) to (48,2), and (49,2) to (64,2) arranged in a second right column of each of the transducer blocks 510, 520, 530, and 540 in order to generate four analog signals a5, a6, a7, and a8, respectively.

The first beamforming unit 710 in the analog beamformer 700 synthesizes signals which are received by 16 transducers (1,3) to (16,3) arranged in a third right column of the first transducer block 510 and processed by the received signal processor 650 based on respective delay signal values t1 to t16 for analog beamforming used for forming a transmission beam in order to generate an analog signal a9. In addition, the second beamforming unit 720 synthesizes signals which are received by 16 transducers (17,3) to (32,3) arranged in a third right column of the second transducer block 520 and processed by the received signal processor 650 based on respective delay signal values t17 to t32 for analog beamforming used for forming a transmission beam in order to generate an analog signal a10. In this manner, the first, second, third, and fourth beamforming units 710, 720, 730, 740 in the analog beamformer 700 synthesize signals which are received by the transducers (1,3) to (16,3), (17,3) to (32,3), (33,3) to (48,3), and (49,3) to (64,3) arranged in a third right column of each of the transducer blocks 510, 520, 530, and 540 in order to generate four analog signals a9, a10, a11, and a12, respectively.

The first beamforming unit 710 in the analog beamformer 700 synthesizes signals which are received by 16 transducers (1,4) to (16,4) arranged in a fourth right column of the first transducer block 510 and processed by the received signal processor 650 based on respective delay signal values t1 to t16 for analog beamforming used for forming a transmission beam in order to generate an analog signal a13. In addition, the second beamforming unit 720 synthesizes signals which are received by 16 transducers (17,4) to (32,4) arranged in a fourth right column of the second transducer block 520 and processed by the received signal processor 650 based on respective delay signal values t17 to t32 for analog beamforming used for forming a transmission beam in order to generate an analog signal a14. In this manner, the first, second, third, and fourth beamforming units 710, 720, 730, 740 in the analog beamformer 700 synthesize signals which are received by the transducers (1,4) to (16,4), (17,4) to (32,4), (33,4) to (48,4), and (49,4) to (64,4) arranged in a fourth right column of each of the transducer blocks 510, 520, 530, and 540 in order to generate four analog signals a13, a14, a15, and a16, respectively.

The analog adder 750 in the analog beamformer 700 adds signals output from the first, second, third, and fourth beamforming units 710, 720, 730, 740 in order to generate four analog signals b1, b2, b3, and b4. In particular, b1=a1+a2+a3+a4, b2= a5+a6+a7+a8, b3=a9+a10+a11+a12, and b4=a13+a14+a15+a16.

The ADC 660 converts the four analog signals b1, b2, b3, and b4 into four digital signals c1, c2, c3, and c4, respectively.

The reception digital beamformer 820 synthesizes the four digital signals c1, c2, c3, and c4 based on respective time delay values d1, d2, d3, and d4 for digital beamforming, calculated by the sub-transducer arrays 550, 560, 570, and 580, in order to generate one or more digital signals e1. The digital signals e1 generated by the reception digital beamformer 820 indicate a focal point of an object or information which relates to the object as a target of an image. A diagnosis image may be generated based on the digital signals e1.

As is apparent from the above description, an apparatus and method for beamforming divides 1 D or 2D array transducers into a plurality of blocks, calculates delay profiles (i.e., delay times) for the respective blocks, and performs beamforming on signals which are processed by each block based on the corresponding calculated delay profile in order to reduce a DDR, thereby reducing a size of a delay line (which size corresponds to an overall size of an analog beamformer).

Although a few exemplary embodiments have been shown and described, it will be appreciated by those skilled in the art that changes may be made in these exemplary embodiments without departing from the principles of the present inventive concept, the scope of which is defined in the claims.

## Claims

1. A beamforming apparatus for beamforming signals which are transmitted or received by a probe which comprises a plurality of transducer blocks, each of the plurality of transducer blocks comprising a respective plurality of transducers, the beamforming apparatus comprising:
a controller configured to control components to delay signals to be transmitted by the plurality of transducers for each of the plurality of transducer blocks and signals received by the plurality of transducers for each of the plurality of transducer blocks; and
an analog beamformer which comprises a plurality of beamforming modules which correspond to the respective transducer blocks and which are configured to perform analog beamforming on signals which are transmitted or received by the corresponding plurality of transducers based on a control signal which is received from the controller.

2. The beamforming apparatus according to claim 1, wherein the controller is further configured to calculate a delay profile for each of the plurality of transducer blocks, to calculate a delay time for each transducer with respect to each of the plurality of transducer blocks based on the corresponding calculated delay profile, and to control the components to delay the signals to be transmitted and the received signals based on the calculated delay times.

3. The beamforming apparatus according to claim 1 or 2, wherein each of the plurality of transducers included in each of the plurality of transducer blocks is one-dimensionally (1 D) arranged or two-dimensionally (2D) arranged.

4. The beamforming apparatus according to any one of claims 1 to 3, further comprising a storage configured to store a number of the transducer blocks and combinations of the plurality of transducers included in each of the plurality of transducer blocks, wherein the transducers included in each of the plurality of transducer blocks are arranged to be adjacent to each other.

5. The beamforming apparatus according to any one of claims 1 to 4, wherein each of the plurality of beamforming modules comprises:
a signal delayer configured to delay signals to be transmitted by the plurality of transducers included in the corresponding transducer block and signals received by the plurality of transducers included in the corresponding transducer block;
a switcher configured to perform a switching operation based on whether signals which are input into the signal delayer are the signals to be transmitted or the received signals; and
an adder configured to add delayed signals which are output from the signal delayer when the signals which are input into the signal delayer are the received signals.

6. The beamforming apparatus according to claim 5, wherein the signal delayer comprises a plurality of delay lines configured to implement respective delay times based on the control signal; and/or
wherein the analog beamformer further comprises an analog adder configured to add signals which are output from the respective adders included in the plurality of beamforming modules,
in particular, further comprising an analog-digital converter configured to convert an analog signal generated by the analog adder into a digital signal.

7. A beamforming apparatus according to claim 1, wherein the transducers are two-dimensional (2D) transducers,
wherein the analog beamformer configured to perform analog beamforming on each of the plurality of transducer blocks in a first direction, each of the plurality of transducer blocks comprising a respective plurality of transducer sub-blocks which is arranged in the first direction, and each of the plurality of transducer sub-blocks comprising a respective plurality of transducers; the apparatus further comprising
a digital beamformer configured to perform digital beamforming in a second direction which is perpendicular to the first direction;
wherein the controller is configured to calculate a delay profile for any one from among the plurality of transducer sub-blocks included in a corresponding transducer block, to calculate a delay time for each transducer included in the corresponding transducer block based on the calculated delay profile, and to control components to delay signals to be transmitted by the plurality of transducers included in the corresponding transducer sub-block and signals received from the plurality of transducers included in the corresponding transducer block based on the calculated delay times.

8. The beamforming apparatus according to claim 7, wherein the controller is further configured to control the components to apply a same first delay time for digital beamforming to transducers which are arranged in a first direction in which the analog beamforming is performed, and to apply a same second delay time for analog beamforming to transducers which are arranged in a second direction in which the digital beamforming is performed.

9. The beamforming apparatus according to claim 7 or 8, wherein the analog beamformer is further configured to add analog-beamformed signals for the respective transducer blocks with respect to the received signals.

10. The beamforming apparatus according to any one of claims 7 to 9, wherein the analog beamformer comprises a plurality of beamforming modules which correspond to the respective transducer blocks, each of the plurality of beamforming modules being configured to perform analog beamforming on signals which are transceived by the plurality of transducers included in the corresponding transducer block based on a control signal received from the controller;
in particular, wherein each of the plurality of beamforming modules comprises:
a signal delayer configured to delay signals to be transmitted by the plurality of transducers included in the corresponding transducer sub-block and signals received by the plurality of transducers included in the corresponding transducer block;
a switcher configured to perform a switching operation based on whether signals which are input into the signal delayer are the signals to be transmitted or the received signals; and
an adder configured to add delayed signals which are output from the signal delayer when the signals which are input into the signal delayer are the received signals;
in particular, wherein the analog beamformer further comprises an analog adder configured to add signals which are output from the respective adders included in the plurality of beamforming modules.

11. A beamforming method for beamforming signals which are transmitted or received by a probe which comprises a plurality of transducer blocks, each of the plurality of transducer blocks comprising a respective plurality of transducers, the method comprising:
calculating, for each of the plurality of transducer blocks, a delay time for each of the plurality of transducers included in a corresponding one of the plurality of transducer blocks based on a delay profile which relates to the corresponding one of the plurality of transducer blocks; and
performing, for each of the plurality of transducer blocks, analog beamforming on signals transceived by the plurality of transducers included in the corresponding one of the plurality of transducer blocks based on the calculated delay times.

12. The beamforming method according to claim 11, wherein the performing the analog beamforming comprises controlling a respective amount of delay for each of transmitted signals and received signals based on the calculated delay times.

13. The beamforming method according to claim 11 or 12, further comprising adding signals resulting from the performing the analog beamforming with respect to the received signals for each transducer blocks.

14. The beamforming method according to any one of claims 11 to 13, wherein each of the plurality of transducer blocks comprises transducers that are one-dimensionally (1 D) arranged or two-dimensionally (2D) arranged.

15. The beamforming method according to any one of claims 11 to 14, further comprising calculating the delay profile for each of the plurality of transducer blocks.
